# EUROPÄISCHE PATENTSCHRIFT

(11) **EP 1 497 259 B1**
(45) Veröffentlichungstag und Bekanntmachung des Hinweises auf die Patenterteilung: **01.08.2007**
(21) Anmeldenummer: 03724984.4
(22) Anmeldetag: 09.04.2003
(51) Int. Cl.: C07C 263/04, B01J 6/00

(54) **REAKTOR ZUR THERMISCHEN SPALTUNG VON MONO- UND POLYFUNKTIONELLEN CARBAMIDS UREESTERN**
REACTOR FOR THERMALLY CRACKING MONOFUNCTIONAL AND POLYFUNCTIONAL CARBAMATES
REACTEUR DE FISSION THERMIQUE D'ESTERS D'ACIDE CARBAMIDE MONOFONCTIONNELS ET POLYFONCTIONNELS

(30) Priorität: 10.04.2002 DE 10215778
(43) Veröffentlichungstag der Anmeldung: 19.01.2005
(73) Patentinhaber: BASF Aktiengesellschaft, 67056 Ludwigshafen (DE)
(72) Erfinder: STAROSTA, Dieter, 01987 Schwarzheide (DE); PFAB, Peter, 67433 Neustadt (DE); KRASE, Volker, 01979 Lauchhammer (DE); SCHMIDT, Andeas, 01987 Schwarzheide (DE); KLÖTZER, Matthias, 01945 Kroppen (DE); OTTERBACH, Andreas, 3080 Tervuren (BE)
(74) Vertreter: Isenbruck, Günter
(86) Internationale Anmeldenummer: PCT/EP2003/003672
(87) Internationale Veröffentlichungsnummer: WO 2003/084921

(56) Entgegenhaltungen:
- EP-A- 0 524 554
- US-A- 5 087 739

## Beschreibung

Die Erfindung betrifft einen Reaktor zur thermischen Spaltung von mono- und/oder polyfunktionellen Carbamidsäureestern in der Flüssigphase sowie ein Verfahren zur thermischen Spaltung von Carbamidsäureestern in einem Reaktor.

Die thermische Spaltung von Carbamidsäureestern zur Gewinnung von Isocyanaten ist seit langem bekannt. Problematisch ist hierbei, dass die Spaltung reversibel ist, d. h., dass die Spaltprodukte Isocyanat- und Hydroxylkomponente beim Abkühlen der heißen Reaktionsgemische erneut rekombinieren. Zur Verhinderung der Rückreaktion ist es erforderlich, durch geeignete verfahrentechnische Maßnahmen die Isocyanat- und Hydroxylkomponenten aus dem Reaktionsgemisch sofort abzutrennen.

Ein weiteres Problem bei der thermischen Spaltung von Carbamidsäureestern ist der Eduktverlust durch eine Vielzahl von irreversiblen Zersetzungsreaktionen der Carbamidsäureester. Es wurden daher Verfahren entwickelt, um dieselben zurückzudrängen, wobei insbesondere die Reaktionstemperatur durch Einsatz von Katalysatoren reduziert wird. Geeignet sind basische Katalysatoren, wie beispielsweise in US 2,692,275 beschrieben, sie beschleunigen jedoch auch die Zersetzungsreaktionen. Die in DE-AS 26 35 490 beschriebenen Aluminium-, Zink- und Zinnverbindungen sollen selektiver sein, und bevorzugt die Spaltungsreaktion beschleunigen.

Als weitere Verfahrensverbesserung, insbesondere für die Herstellung polyfunktioneller Isocyanate, bei denen die Gefahr der Polymerisation und Rückstandsbildung verstärkt besteht, wurde die Verdünnung der Carbamidsäureester mit inerten Lösungsmitteln bzw. das Zudosieren von inerten Lösungsmitteln in den Spaltreaktor vorgeschlagen, beispielsweise in EP-A-0 092 738. Durch diese Maßnahmen kann die Verschmutzung der Flächen im Reaktor zurückgedrängt werden und es können Nebenprodukte ausgeschleust werden. Nachteilig an einem solchen Zusatz von Hilfsmitteln sind der daraus resultierende erhöhte verfahrenstechnische Aufwand bzw. die Kosten für den Verbrauch der Hilfsmittel.

Bekannt ist auch der Einsatz von katalytisch wirksamen Lösungsmitteln, beispielsweise von Dialkylanilinen, wie in US 4,294,774 vorgeschlagen.

Zu apparativen Ausgestaltung des Spaltungsrealctors sind eine Vielzahl von Vorschlägen bekannt. Die Spaltungsreaktoren müssen den oben beschriebenen Besonderheiten der Carbamidspaltungsreaktion Rechung tragen, insbesondere die Rekombination der Spaltungsprodukte sowie Nebenreaktionen unterdrücken. Dazu ist es erforderlich, die Verweilzeit der Carbamidsäureester in der heißen Zone des Spaltreaktors sehr kurz zu halten und die Spaltprodukte rasch aus dem Reaktionsraum zu entfernen. Als hierfür geeignete Spaltreaktoren werden in der EP-A 0 092 738 Dünnschichtverdampfer und Fallfilmverdampfer beschrieben. Derartige Reaktoren sind jedoch für den Einsatz im großtechnischen Maßstab teuer und die Dünnschichtverdampfer haben darüber hinaus den Nachteil, dass sie mit einem Rührwerk ausgestattet sein müssen, d. h. dass sie bewegte Teile aufweisen. Der größte Nachteil derartiger Reaktoren besteht darin, dass sie bei der Spaltung polyfunktioneller Carbamidsäureester durch Polymerisation verschmutzen, wenn sie über einen längeren Zeitraum als einige Tage betrieben werden, ein wirtschaftlicher Betrieb somit nicht möglich ist.

Die EP-A 0 524 554 beschreibt den Einsatz von einfachen Kesseln als Spaltreaktoren, die Einbauten zur Einbringung der Reaktionswärme enthalten. Dabei wird durch die Geometrie des Reaktors, gekennzeichnet durch die Entgasungsfläche im Verhältnis zum Volumen sowie durch die Anordnung der Heizflächen, die Spaltung in einem zweiphasigen Gemisch ermöglicht, das über 50% volumetrischen Gasgehalt aufweist. Das Reaktionsmedium liegt in diesen Reaktoren in einem siedeähnlichen Zustand vor, der dadurch gekennzeichnet ist, dass die aus der Spaltreaktion resultierenden Gase und Eduktdämpfe in der Flüssigkeit aufsteigen.

Beispielhaft werden genannt: Robertverdampfer, Herbertverdampfer mit und ohne Zwangsumlauf, "Heizkerzenverdampfer" mit senkrechten, schrägstehenden oder waagrechten Heizkerzen, Caddletype Verdampfer, Kessel mit eng gewickelten Heizschlangen, und ähnliche Reaktoren, die sich durch einen hohen Wärmeeintrag in ein kleines Volumen auszeichnen.

Derartige Reaktoren lassen sich bei Einhaltung der beschriebenen Randbedingungen so betreiben, dass auch mehrfunktionelle Carbamidsäureester zu den entsprechenden Isocyanat- und Hydroxylkomponenten gespalten werden können, ohne dass die Reaktoren im Verlauf einer mehrwöchigen, kontinuierlichen Betriebszeit abgestellt und gereinigt werden müssen.

Bei der häufig eingesetzten Variante als Rohrbündelreaktor, mit Durchleitung des Wärmetauschmittels durch die Rohre und des Reaktionsmediums durch den Zwischenraum zwischen den Rohren des Rohrbündels, besteht das Problem, dass im Bereich der Schweißnähte zur Befestigung der Rohre in Rohrböden an beiden Reaktorenden thermische Spannungen auftreten mit Auswirkungen auf die Dichtflächen, mit der Folge von Undichtigkeiten bei An- und Abfahrvorgängen sowie Produktanbackungen in diesem Reaktorbereich.

Demgegenüber war es Aufgabe der Erfindung, einen Reaktor mit Einrichtungen zur Einbringung von Reaktionswärme mit erhöhten Standzeiten von mehreren Monaten zur Verfügung zu stellen, der einfach und kostengünstig herstellbar und bei Bedarf einfach erweiterbar ist, und der die oben aufgezeigten Nachteile nicht aufweist.

Die Lösung geht aus von einem Reaktor zur thermischen Spaltung von mono- und/oder polyfunktionellen Carbamidsäureestern in die entsprechenden Isocyanat- und Hydroxylkomponenten in der Flüssigphase ohne Zusatz eines Lösungsmittels, mit Einrichtungen zum Wärmeeintrag in den Reaktor, dessen Geometrie, gekennzeichnet durch die Entgasungsfläche im Verhältnis zum Volumen der Flüssigphase und die Anordnung der Heizflächen, die Spaltung in einem zweiphasigen Gemisch ermöglicht, das über 50 Vol.-% Gasgehalt aufweist.

Die Erfindung ist dadurch gekennzeichnet, dass die Einrichtungen von einem Wärmetauschmittel durchströmte Wärmetauscherplatten sind.

Der erfindungsgemäße Reaktor wird bei Verfahrensbedingungen betrieben, wie sie in EP-A 0524 554 beschrieben sind. Der Reaktor wird so betrieben, dass die zweiphasige Mischung aus siedender Flüssigkeit und Dampfphase, die aus dem verdampften Edukt und den Spaltgasen besteht, über 50% volumetrischen Gasanteil enthält.

Der volumetrische Gasgehalt der zweiphasigen Mischung soll bevorzugt zwischen 50 und 98%, vorzugsweise zwischen 60 und 96% und besonders bevorzugt zwischen 75 und 90% liegen.

Zur Realisierung derart hoher Gasgehalte in der zweiphasigen Mischung sind bestimmte geometrische Anforderungen an die Reaktoren sinnvoll.

So ist es beispielsweise sinnvoll, die Höhe der Reaktionszone im Reaktor nicht kleiner als 0,2 m und nicht größer als 2 m zu wählen, um die Ausbildung der gasreichen zweiphasigen Mischung zu fördern. Die Entgasungsfläche des Reaktors, das heißt die freie Oberfläche der Flüssigkeit, an der Gas entweichen kann, sollte so bemessen sein, dass die Gasgeschwindigkeit am oberen Ende der Reaktionszone nicht unter 1 m/s und nicht über 30 m/s, bevorzugt zwischen 2 m/s und 20 m/s, beträgt. Die Wärmeübertnagungsflächen sollten so dimensioniert sein, dass die Temperaturdifferenz zwischen Heiz- und Reaktionsmedium kleiner als 40°C ist, aber gleichzeitig die für die stark endotherme Reaktion erforderliche Wärmemenge in das durch Verweilzeitanforderungen beschränkte Volumen eingebracht werden kann.

Die Reaktoren werden vollkontinuierlich betrieben, sowohl hinsichtlich der Produktzufuhr als auch hinsichtlich einer Entnahme von Flüssigkeit, um die Anreicherung höhersiedender Nebenprodukte zu vermeiden. Der Umsatz im Reaktor pro Durchgang beträgt zwischen 30 und 95%, vorzugsweise zwischen 60 und 90% Umwandlung von Urethan im Reaktorzulauf in entnommenes Isocyanat.

Die mittleren Verweilzeiten betragen 1 bis 80 Minuten, bevorzugt 5 bis 60 Minuten und sind definiert als der Quotient aus dem flüssigen Inhalt des Reaktors unter Reaktionsbedingungen und dem pro Minute aus dem Reaktor flüssig entnommenen Reaktorinhalt.

Die Reaktion wird ohne jeden Zusatz von Löse- oder Verdünnungsmittel durchgeführt und ohne dass ein Inertgas durch den Reaktor gefahren wird.

Dem Reaktor wird ein Produktgemisch zugeführt, das aus dem Carbamidsäureester und aus Nebenprodukten besteht, die in einem Kreislaufverfahren entstehen, das dadurch gekennzeichnet ist, dass aus einem Amin unter Zugabe von Harnstoff und einer Hydroxylkomponente ein Carbamidsäureester hergestellt wird, der dann zum Isocyanat gespalten wird, wobei ein Teil des Reaktorinhalts dem Reaktor entnommen und wieder in die erste Stufe des Verfahrens zurückgefahren wird. Solche Nebenprodukte können unter anderem hochmolekulare Produkte, vorzugsweise Isocyanurate, sein. Überraschenderweise genügt es, wenn der Gehalt an Carbamidsäureester im Reaktorzulauf zwischen 80 und 90% beträgt. Eine höhere Reinheit des Carbamidsäureesters erleichtert den Betrieb des Spaltreaktors, da dann Foulingprobleme an Bedeutung verlieren. Es können auch Reaktorzuläufe mit weniger als 80% Carbamidsäureester verwendet werden, aber Foulingprobleme nehmen dann zu, insbesondere wenn mehrfunktionelle Carbamidsäureester gespalten werden. Der Spaltreaktorzulauf kann gegebenenfalls den für die Spaltung erforderlichen Katalysator enthalten.

Wesentlich für die Erfindung ist die Tatsache, dass der Carbamidsäureester, bevor er dem Reaktor zugeführt wird, nicht komplett verdampft und wieder kondensiert werden muss, um ihn von den oligomeren Nebenprodukten zu befreien, wie es in der EP-A 355 433 beschrieben wird, sondern dass ein gewisser Pegel an Nebenprodukten im Reaktor toleriert werden kann.

Indem erfindungsgemäß Wärmetauscherplatten als Einrichtungen zum Eintrag von Wärme eingesetzt werden, ist es möglich, auch Carbamidsäureester mit einem höheren Grad an Verunreinigungen, und zwar mit einem Gehalt an Carbamidsäureester von 70 bis 85%, zu spalten.

Erfindungsgemäß werden im Spaltreaktor als Einrichtungen zum Wärmeeintrag Wärmetauscherplatten eingesetzt.

Wärmetauscherplatten sind in der Verfahrenstechnik bekannte Einrichtungen zur Wärmeübertragung. Sie sind in der Regel aus zwei im wesentlichen parallel zueinander ausgerichteten Blechen gebildet, die miteinander verbunden, insbesondere verschweißt sind, und einen Zwischenraum ausbilden, der über geeignete Zu- und Abführleitungen von einem Wärmetauschmittel durchströmt werden kann. Zur Erhöhung der Stabilität, insbesondere der Druckfestigkeit, sind die Platten häufig an einer Vielzahl von Stellen miteinander punkt- und/oder längsverschweißt.

Wärmetauscherplatten werden in der Regel in einem Stapel, d. h. in einer Vielzahl von parallel zueinander angeordneten Wärmetauscherplatten eingesetzt. Für den erfindungsgemäßen Reaktor werden die Wärmetauscherplatten bevorzugt aus Edelstahl gefertigt, insbesondere aus einem Edelstahl mit den Werkstoffnummern 1.45xx, wobei die letzten beiden, jeweils mit x bezeichneten Ziffern beliebig sein können, vorzugsweise aus einem Edelstahl mit einer der Werkstoffnummern 1.4571, 1.4529, 1.4401, 1.4404 oder 1.4462. Die Wärmetauscherplatten werden bevorzugt mit glatter, insbesondere polierter, beispielsweise elektropolierter Oberfläche ausgebildet.

Durch ihre geometrische Ausgestaltung zwingen die Wärmetauscherplatten dem in den Zwischenräumen zwischen den Wärmetauscherplatten strömendem Reaktionsgemisch eine Strömung in Längsrichtung auf. Dadurch ist der Wärmeaustausch mit dem die Wärmetauscherplatten durchströmenden Wärmetauschmittel schlechter gegenüber einem Rohrbündelreaktor, in dem auch eine Quervermischung des Reaktionsmediums zwischen den von Wärmetauschmitteln durchströmten Rohren möglich ist. Daher ist bei gleichem Reaktionsvolumen für einen Spaltreaktor mit Wärmetauscherplatten eine größere Wärmetauschfläche gegenüber einem Spaltreaktor mit einem Rohrbündel erforderlich. So muss beispielsweise eine Wärmetauscherfläche von 150 m² für einen Rohrbündelreaktor auf 228 m² für einen Reaktor mit Wärmetauscherplatten erhöht werden, bei gleichem Reaktionsvolumen. Da der Plattenreaktor jedoch fertigungstechnisch einfacher herzustellen ist, liegen die Fertigungskosten dennoch bei lediglich 25 bis 30% der Fertigungskosten für den Rohrbündelreaktor.

Darüber hinaus kann bei Einsatz von Wärmetauscherplatten aufgrund der erhöhten Wärmeträgerfläche bei gleichem Reaktionsvolumen die Wärmeträgervörlauftemperatur und damit die Heizflächenbelastung abgesenkt werden, häufig um bis zu ca. 20°C. Dies führt zu einer geringeren Belagbildung an den Oberflächen und damit zur Verlängerung der Reaktorstandzeit, häufig um ca. 20%.

Der erfindungsgemäße Spaltreaktor ist bevorzugt dergestalt ausgebildet, dass die Wärmetauscherplatten in die Flüssigphase und das zweiphasige Reaktionsgemisch teilweise oder vollständig eintauchen.

Als Wärmetauschmittel in den Wärmetauscherplatten werden bevorzugt hochsiedende Flüssigkeiten, d. h. Flüssigkeiten mit einem Siedepunkt von mindestens ca. 40°C oberhalb der Spaltungstemperatur der eingesetzten Carbamidsäureester, insbesondere mit einem Siedepunkt im Bereich von 280 bis 400°C, bevorzugt von 350 bis 390°C eingesetzt. Besonders bevorzugt wird als Wärmetauschmittel ein Marlothermöl^{®} oder Gemische von Marlothermölen^{®} eingesetzt. Marlotherme^{®} sind Gemische isomerer Dibenzyltoluole (Marlotherm^{®} S) oder Gemische isomerer Benzyltoluole (Marlotherm^{®} L).

Die Wärmetauscherplatten werden bevorzugt dergestalt dimensoniert, dass das Verhältnis zwischen der gesamten Wärmetauschfläche der Wärmetauschplatten und dem Gesamtvolumen der Flüssigphase im Reaktor im Bereich von 10 bis 320 m²/m³, bevorzugt im Bereich von 20 bis 150 m²/m³, insbesondere bevorzugt im Bereich von 50 bis 80 m²/m³ liegt.

Der Spaltreaktor ist in einer bevorzugten Ausführungsform ein liegender Zylinder, wobei die Wärmetauscherplatten als parallel zueinander und senkrecht zur Reaktorlängsrichtung in der unteren Reaktorhälfte angeordnete Kreissegmente ausgebildet sind.

Besonders bevorzugt sind die Kreissegmente kleiner als die Hälfte des Reaktorquerschnitts.

Es ist bevorzugt, die Halterungen der Wärmetauscherplatten möglichst schwingungsfrei auszubilden, um die Turbulenz im Reaktor nicht zu stören. Hierfür werden die Wärmetauscherplatten bevorzugt an Zu- und Abführungsrohren, die in Reaktorlängsrichtung oberhalb der Wärmetauscherplatten angeordnet sind, mittels bogenförmiger Rohrstücke gehalten.

Durch diese besondere Ausbildung der Zu- und Abführung für das Wärmetauschmittel gibt es im Spaltreaktor in den Hauben an beiden Enden desselben, im Bereich der Zu- bzw. Abführung des Wärmetauschmittels keine großen, erwärmten Flächen mehr, an denen Anbackungen von Nebenprodukten stattfinden könnten, wie dies auf den beheizten Rohrböden von Rohrbündelreaktoren der Fall ist.

Darüber hinaus kann der erfindungsgemäße Spaltreaktor mit Wärmetauscherplatten in einfacher Weise an einen erhöhten Bedarf an Wärmetauschfläche durch Anbringen zusätzlicher Heizplatten und Erweiterung des Reaktormantels angepasst werden.

Gegenstand der Erfindung ist auch ein Verfahren zur thermischen Spaltung von Carbamidsäureestern in einem oben beschriebenen Spaltreaktor.

Bezüglich der einsetzbaren mono- oder polyfunktionellen Carbamidsäureester gibt es grundsätzlich keine Einschränkungen.

Das Verfahren ist auch nicht eingeschränkt bezüglich der weiteren Aufarbeitung der bei der Spaltreaktion aus dem Reaktor entweichenden Spaltgase. Die Auftrennung derselben kann bevorzugt rektifikativ erfolgen, wie in EP-A 0 524 554 beschrieben.

Bevorzugt wird das Verfahren in der Weise durchgeführt, dass dem Reaktionsmedium kein Lösungsmittel zugesetzt wird.

Der Spaltreaktor wird bevorzugt bei einem Druck von 2 bis 200 mbar, besonders bevorzugt bei einem Druck von 5 bis 100 mbar betrieben.

Der Spaltreaktor wird bevorzugt in der Weise betrieben, dass die Geschwindigkeit der entweichenden Gase am oberen Ende der Reaktionszone 1 bis 30 m/s, bevorzugt 2 bis 20 m/s beträgt, wenn man die Reaktion bei einem absoluten Druck zwischen 2 mbar und 200 mbar durchführt.

Die Erfindung wird im folgenden anhand einer Zeichnung sowie eines Ausführungsbeispiels näher erläutert.

Es zeigt:

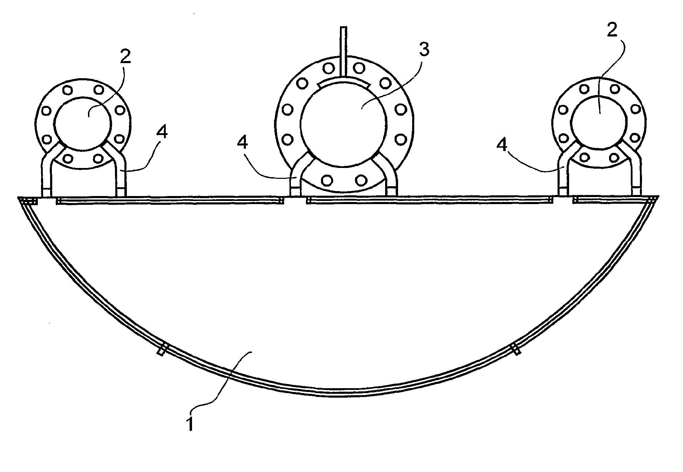

- Figur 1: die schematische Darstellung einer bevorzugten Ausführungsform einer Wärmetauscherplatte im Querschnitt.

Figur 1 zeigt eine kreissegmentförmige Wärmetauscherplatte 1. Im oberen Teil der Wärmetauscherplatte 1 sind Verteil- und Sammeleinrichtungen 2, 3 für das Wärmetauschmittel vorgesehen, die beispielhaft als Rohre mit kreisförmigem Querschnitt ausgebildet sind. In der in Figur 1 dargestellten bevorzugten Ausführungsvariante wird das Wärmetauschmittel über die äußeren Verteil- und Sammeleinrichtungen 2 zugeführt und über die innere Verteil- und Sammeleinrichtung 3 abgeführt. Die Verbindung der Verteil- und Sammeleinrichtungen 2, 3 zu den Wärmetauscherplatten 1 erfolgt über bogenförmige Rohrstücke 4, die eine weitgehend schwingungsfreie Halterung gewährleisten. Im erfindungsgemäßen Spaltreaktor sind eine Vielzahl von Wärmetauscherplatten 1 hintereinander angeordnet, mit entsprechenden Verteil- und Sammeleinrichtungen 2, 3 und bogenförmigen Rohrstücken 4. Dadurch können die hintereinander angeordneten bogenförmigen Rohrstücke als kammartige Halterung der Wärmetauscherplatten 1 bezeichnet werden.

Einem liegenden zylindrischen Reaktor mit einem Durchmesser von 40 cm wurde kontinuierlich ein flüssiger Feedstrom von 45 kg/h Hexamethylendi-n-butylurethan zugeführt. Die Spaltreaktion wurde bei 240°C im Reaktionsmedium und 30 mbar Druck durchgeführt. Über eine Pumpe wurde kontinuierlich ein Flüssigkeitsstrom von 15 kg/h vom Boden des Reaktors abgezogen. Der Flüssigkeits-hold-up in Reaktor und Rohrleitungen betrug ca. 10 Liter. Die mittlere Verweilzeit lag im Bereich von 30 bis 40 Minuten. Dem Reaktionsmedium mussten für die endotherme Reaktion sowie zur Verdampfung der Reaktionsprodukte ca. 10 kW an thermischer Leistung zugeführt werden. Als Wärmetauschmittel wurde Marlotherm® S eingesetzt.

Unter den oben genannten Bedingungen wurde zum Vergleich ein Spaltreaktor mit einem Bündel von 15 Rohren mit einem Durchmesser von jeweils 1,8 cm und ein Spaltreaktor nach der Erfindung, mit Wärmetauscherplatten eingesetzt. Im Vergleichsversuch mit dem Rohrbündelreaktor betrug die Vorlauftemperatur für das Marlothermöl^{®} 260°C und die Wärmetauscherfläche ca. 0,35 qm.

Der erfindungsgemäße Reaktor war demgegenüber mit 9 Wärmetauscherplatten, die vertikal im Reaktor mit einem Abstand zwischen den einzelnen Platten von jeweils 15 mm angeordnet waren, durchgeführt. Die Vorlauftemperatur des Marlothermöls^{®} betrug 258°C und die Wärmetauschfläche betrug ca. 0,6 qm.

Sowohl im Reaktor nach dem Stand der Technik als auch im erfindungsgemäßen Reaktor erfolgte die Spaltung der Carbamidsäureester mit gleichem Umsatz von ca. 95%. Chromatographische Untersuchungen zeigten jedoch, dass die Zunahme von hochmolekularen, zu Verschmutzungen führenden Verbindungen, insbesondere von Allophanaten, Cyanuraten usw. beim Einsatz des erfindungsgemäßen Spaltreaktors mit Wärmetauscherplatten um ca. 15% geringer war. Nach drei Wochen Betriebszeit wurden beim erfindungsgemäßen Reaktor im Gegensatz zum Reaktor nach dem Stand der Technik keine Verschmutzungen an den Wärmetauscherflächen festgestellt.

## Patentansprüche

1. Reaktor zur thermischen Spaltung von mono- und/oder polyfunktionellen Carbamidsäureestern in die entsprechenden Isocyanat- und Hydroxylkomponenten in der Flüssigphase ohne Zusatz eines Lösungsmittels, mit Einrichtungen zum Wärmeeintrag in den Reaktor, dessen Geometrie, **gekennzeichnet durch** die Entgasungsfläche im Verhältnis zum Volumen der Flüssigphase und die Anordnung der Heizflächen, die Spaltung in einem zweiphasigen Gemisch ermöglicht, das über 50 Vol.-% Gasgehalt aufweist, **dadurch gekennzeichnet, dass** die Einrichtungen von einem Wärmetauschmittel durchströmte Wärmetauscherplatten (1) sind.

2. Reaktor nach Anspruch 1, **dadurch gekennzeichnet, dass** die Wärmetauscherplatten (1) in die Flüssigphase teilweise oder vollständig eintauchen.

3. Reaktor nach Anspruch 1 oder 2, **dadurch gekennzeichnet, dass** das Wärmetauschmittel eine hochsiedende Flüssigkeit, insbesondere eine. Marlothermöl^{®} oder ein Gemisch von Märlothermölen^{®} ist.

4. Reaktor nach einem der Ansprüche 1 bis 3, **gekennzeichnet durch** ein Verhältnis zwischen der gesamten Wärmetauschfläche der Wärmetauschplatten (1) und dem Gesamtvolumen der Flüssigphase im Reaktor im Bereich von 10 bis 320 qm/m³, bevorzugt im Bereich von 20 bis 150 qm/m³, insbesondere bevorzugt im Bereich von 50 bis 80 qm/m³.

5. Reaktor nach einem der Ansprüche 1 bis 4, **dadurch gekennzeichnet, dass** er als liegender . Zylinder, und dass die Wärmetauscherplatten (1) als in der unteren Reaktorhälfte, parallel zueinander und senkrecht zur Reaktorlängsachse angeordnete Kreissegmente ausgebildet sind.

6. Reaktor nach Anspruch 5, **dadurch gekennzeichnet, dass** die Kreissegmente kleiner als die Hälfte des Reaktorquerschnitts sind, insbesondere einen Anteil von 20 bis 30% der Querschnittsfläche des Reaktors aufweisen.

7. Reaktor nach einem der Ansprüche 1 bis 6, **dadurch gekennzeichnet, dass** die Wärmetauscherplatten (1) eine weitgehend vibrationsfreie Halterung an in Reaktorlängsrichteung, oberhalb der Wärmetauscherplatten angeordneten Zuführungs- und Verteilungsrohren (2, 3) mittels bogenförmiger Rohrstücke (4) aufweisen.

8. Verfahren zur thermischen Spaltung von mono- und/oder polyfunktionellen Carbamidsäureestern in die entsprechenden Isocyanat- und Hydroxylkomponenten in der Flüssigphase ohne Zusatz eines Lösungsmittels, wobei das Verfahren in einem Reaktor mit Einrichtungen zum Wärmeeintrag in den Reaktor durchgeführt wird, dessen Geometrie, **gekennzeichnet durch** die Entgasungsfläche im Verhältnis zum Volumen der Flüssigphase und die Anordnung der Heizflächen, die Spaltung in einem zweiphasigen Gemisch ermöglicht, das über 50 Vol.-% Gasgehalt, aufweist, und wobei die Einrichtungen von einem Wärmetauschmittel durchströmte Wärmetauscherplatten (1) sind.

9. Verfahren nach Anspruch 8, **dadurch gekennzeichnet, dass** man das Verfahren bei einem Druck von 2 bis 200 mbar, bevorzugt bei einem Druck von 5 bis 100 mbar im Reaktor durchführt.

10. Verfahren nach Anspruch 8 oder 9, **dadurch gekennzeichnet, dass** die Geschwindigkeit der entweichenden Gase am oberen Ende der Reaktionszone 1 m/s bis 30 m/s, bevorzugt 2 m/s bis 20 m/s, beträgt.

## Claims

1. A reactor for the thermal dissociation of monofunctional and/or polyfunctional carbamic esters into the corresponding isocyanate and hydroxyl components in the liquid phase without addition of a solvent, having devices by means of which heat can be introduced into the reactor and whose geometry, defined by the ratio of the degassing area to the volume of the liquid phase and the arrangement of the heating surfaces, makes it possible for the dissociation to occur in a two-phase mixture which has a gas content of over 50% by volume, wherein the devices are heat exchanger plates (1) through which a heat transfer medium flows.

2. The reactor according to claim 1, wherein the heat exchanger plates (1) are immersed partly or completely in the liquid phase.

3. The reactor according to claim 1 or 2, wherein the heat transfer medium is a high-boiling liquid, in particular a Marlotherm^{®} fluid or a mixture of Marlotherm^{®} fluids.

4. The reactor according to any of claims 1 to 3 which has a ratio of the total heat exchange area of the heat exchanger plates (1) to the total volume of the liquid phase in the reactor in the range from 10 to 320 m²/m³, preferably in the range from 20 to 150 m²/m³, particularly preferably in the range from 50 to 80 m²/m³.

5. The reactor according to any of claims 1 to 4 which is configured as a horizontal cylinder and in which the heat exchanger plates (1) are configured as segments of a circle which are arranged parallel to one another and perpendicular to the longitudinal axis of the reactor in the lower half of the reactor.

6. The reactor according to claim 5, wherein the segments of a circle are smaller than half the cross section of the reactor and in particular correspond to a proportion of from 20 to 30% of the cross-sectional area of the reactor.

7. The reactor according to any of claims 1 to 6, wherein the heat exchanger plates (1) are mounted in a largely vibration-free manner by means of arc-shaped pipe sections (4) on feed pipes and distribution pipes (2, 3) arranged in the longitudinal direction of the reactor above the heat exchanger plates.

8. A process for the thermal dissociation of monofunctional and/or polyfunctional carbamic esters into the corresponding isocyanate and hydroxyl components in the liquid phase without addition of a solvent, wherein the process is carried out in a reactor having devices by means of which heat can be introduced into the reactor and whose geometry, defined by the ratio of the degassing area to the volume of the liquid phase and the arrangement of the heating surfaces, makes it possible for the dissociation to occur in a two-phase mixture which has a gas content of over 50% by volume and the devices are heat exchanger plates (1) through which a heat transfer medium flows.

9. The process according to claim 8 carried out at a pressure of from 2 to 200 mbar, preferably at a pressure of from 5 to 100 mbar, in the reactor.

10. The process according to claim 8 or 9, wherein the velocity of the gases leaving the upper end of the reaction zone is from 1 m/s to 30 m/s, preferably from 2 m/s to 20 m/s.

## Revendications

1. Réacteur pour la décomposition thermique d'esters mono- et / ou polyfonctionnels d'acide carbamique en les composants isocyanato et hydroxylés correspondants, en phase liquide, sans addition d'un solvant, avec des dispositifs d'apport de chaleur dans le réacteur, dont la géométrie, **caractérisée par** la surface de dégazage par rapport au volume de la phase liquide et l'agencement des surfaces de chauffe, permet la décomposition en un mélange à deux phases présentant une teneur en gaz de plus de 50% en volume, **caractérisé en ce que** les dispositifs sont des plaques d'échangeur de chaleur (1) parcourues par un milieu d'échange de chaleur.

2. Réacteur suivant la revendication 1, **caractérisé en ce que** les plaques d'échangeur de chaleur (1) sont partiellement ou totalement immergées dans la phase liquide.

3. Réacteur suivant la revendication 1 ou 2, **caractérisé en ce que** le milieu d'échange de chaleur est un liquide à haut point d'ébullition, en particulier une huile Marlotherm® ou un mélange d'huiles Marlotherm®.

4. Réacteur suivant l'une quelconque des revendications 1 à 3, **caractérisé par** un rapport entre la surface totale d'échange de chaleur des plaques d'échangeur de chaleur (1) et le volume total de la phase liquide dans le réacteur est de l'ordre de 10 à 320 m²/m³, de préférence de l'ordre de 20 à 150 m²/m³, plus préférablement de l'ordre de 50 à 80 m²/m³.

5. Réacteur suivant l'une quelconque des revendications 1 à 4, **caractérisé en ce qu'**il est configuré en cylindre couché, et que les plaques d'échangeur de chaleur (1) sont configurées en segments de cercle disposés dans la moitié inférieure du réacteur parallèlement entre eux et perpendiculairement à l'axe longitudinal du réacteur.

6. Réacteur suivant la revendication 5, **caractérisé en ce que** les segments de cercle sont plus petits que la moitié de la section transversale du réacteur, et représentent en particulier une fraction de 20 à 30% de la surface de la section transversale du réacteur.

7. Réacteur suivant l'une quelconque des revendications 1 à 6, **caractérisé en ce que** les plaques d'échangeur de chaleur (1) présentent une fixation pratiquement exempte de vibrations agencée à des tubes d'amenée et de distribution (2,3) disposés au-dessus des plaques d'échangeur de chaleur, dans la direction longitudinale du réacteur, au moyen de pièces tubulaires (4) en forme d'arceaux.

8. Procédé de décomposition thermique d'esters mono- et / ou polyfonctionnels d'acide carbamique en les composants isocyanato et hydroxylés correspondants, en phase liquide, sans addition d'un solvant, le procédé étant entrepris dans un réacteur équipé de dispositifs pour l'apport de chaleur dans le réacteur, et dont la géométrie, **caractérisée par** la surface de dégazage par rapport au volume de la phase liquide et l'agencement des surfaces de chauffe permet la décomposition en un mélange à deux phases, qui présente une teneur en gaz de plus de 50% en volume, et où les dispositifs sont des plaques d'échangeur de chaleur (1) parcourues par un milieu d'échange de chaleur.

9. Procédé suivant la revendication 8, **caractérisé en ce que** l'on entreprend le procédé sous une pression de 2 à 200 mbar, de préférence sous une pression de 5 à 100 mbar dans le réacteur.

10. Procédé suivant la revendication 8 ou 9, **caractérisé en ce que** la vitesse du gaz qui se dégage à l'extrémité supérieure de la zone de réaction est de 1 m/s à 30 m/s, de préférence de 2 m/s à 20 m/s.
